Europäisches Patentamt

European Patent Office

Office européen des brevets

(19)

(11) Numéro de publication: **0 473 861 A1**

## (12) DEMANDE DE BREVET EUROPEEN

(21) Numéro de dépôt: 90810663.6

(22) Date de dépôt: 03.09.90

(51) Int. Cl.⁵: **A61N 1/40**, A61N 1/18,
A61N 1/08, A61N 5/06

(43) Date de publication de la demande:
**11.03.92 Bulletin 92/11**

(84) Etats contractants désignés:
**AT BE CH DE DK ES FR GB GR IT LI LU NL SE**

(71) Demandeur: **Moretti, Francesco Avv.**
**Via Peri 2/A, c/o Studio Legale**
**CH-6900 Lugano(CH)**

(72) Inventeur: **Mosca Tenna, Giovanni**
**Elektrastrasse 38**
**W-8000 München 81(DE)**

(74) Mandataire: **Moretti, Francesco, Dr.**
**e/o ENGIMPEX S.A., Industrial Property Serv.**
**Dept., Via Ponte Tresa 7b, P.O. Box 12**
**CH-6924 Sorengo(CH)**

(54) **Procédé et appareil pour le traitement de l'information engendrée par l'application de champs électriques et électromagnétiques à un être vivant.**

(57) Des champs électriques de basse et infrabasse fréquence et des champs électro-magnétiques cohérents de très basse puissance, émis par deux sources laser à paramètres d'excitation différents, sont appliqués à un être vivant dans le but d'obtenir des effets thérapeutiques différents. Le procé vivant est realisé par l'intermédiaire d'une interface sensitive-excitatrice disposée dans un montage spatial interférentiel au niveau des zones dermiques actives, préalablement sélectionnées et caractérisées en base de mensurations des propriétés électrocinétiques.

Le système présente une architecture réalisée par des sous-ensembles d'exécution spécialisée, coordonnés et contrôlés par un système expert. Le système expert est prevu avec des bases de données et de connaissances dynamiques dans lesquelles il y a des sets de paramètres d'application correspondentes, établis précédemment par des méthodes expérimentales et statistiques.

EP 0 473 861 A1

EP 0 473 861 A1

Fig. 1

1. INTRODUCTION

Dans le cas des divers processus pathologiques humains se produisent des modifications de la polarité tissulaire affectée. Ces modifications causent l' apparition d'un gradient électrochimique qui établi un "courant lesionnaire". Ce courant se dirige au niveau de la surface cutanée par des voies de résistance électrique minimale, simultanément avec la propagation d'un grasient thermique qui se produit entre la lesion et le tissu sain adjacent, par l'intermédiaire du système vasculaire. La propagation de ces modifications se produit par les moyens de corrélation viscéro- et somato-cutanée au niveau des surfaces de projection cutanée. Celles-ci présentent des liaisons fonctionnelles de type local, segmentaire et dermatomique dans les zones réceptrices corticales et thalamiques, integrées dans des circuits régulateurs corticaux/subcorticaux. Au niveau des surfaces de projection cutanée le courant lesionnaire cause la modification des moments de dipole et de la configuration électrocinétique de surface. En même temps l'accroissement local de la température cutanée déterminée par la propagation du gradient thermique lesionnaire produit une variation linéaire de la conductibilité électrique et, par conséquence, des modifications synchroniques du potentiel. L' identification, la cartation anatomotopographique et la caractérisation fonctionnelle bioélectrique et thermique de ces surfaces de projection cutanée des modifications électrochimiques et thermodynamiques, survenues dans le cas des divers processus pathologiques, peuvent présenter réellement des valences diagnostiques et de monitorisation des procedures stimulo-thérapeutiques appliquées en but curatif.

Le champ électrique aux diverses fréquences et amplitudes stimule la prolifération cellulaire, DNA, RNA et la biosynthèse protéique, la régénération osseuse, etc.. Ces effets sont dus au fait que les variations du champ électrique sont des signaux utilisés dans la communication inter-cellulaire, inter-tissulaire et inter-organisme.

Au niveau infra-cellulaire les ptotéines de membrane et les systèmes enzymatiques, présentant des charges électriques permanentes et des moments de dipole, sont capable de répondre aux signaux électriques, en traduisant l'énergie libre contenue dans le champ électrique en réactions chimiques endo-ergoniques. L' énergie libre peut être transférée des champs électriques non-stationnaires aussi pour la formation et le maintien des gradients de concentration le long des membranes cellulaires. L'application du champ électrique conduit aussi aux modifications dans le transport des substances d'une part et de l'autre de ceci.

Les effets du champ électromagnétique sur les organismes vivants sonr dépendants du type du champ, de l'intensité, du gradient, du vecteur, de la fréquence, de la forme de l'impulsion, de la localisation, du temps d'exposition, de la composition (électrique, magnétique). L'existence de "fenêtres" pour les divers types de champs électromagnétiques amplifie les propriétés du système biologique de transfert d'énergie libre, propriétés qui sont "coopératives" et "loin de l'équilibre".

Dans les systèmes biomoléculaires la modulation du flux des signaux et la transmission de l'énergie des champs électromagnétiques appliqués se produisent initialement sur le plan atomique, suivies par des modifications de conformation et par des réactions chimique, la variation de l'excitant électromagnétique ayant ainsi des diverses significations d'information pour la biostructure ou le système biologique.

La conduite tempo-spatiale de l'information dans le système biologique s'effectue sans consommation d'énergie mais chaque manipulation d'information implique un apport materiel et un apport énergétique. Les modifications bioénergétiques de nature d'information présentent une magnitude extrêmement basse, amplifiée et en état de détection au niveau des "fenêtres" situées aux niveaux des structures d'enveloppement des organismes. Ces "fenêtres" se modifient dans le sens énergodynamique ascendant ou descendant au transfert d'énergie. Grâce à ce fait, on a constaté expérimentalement que des stimuli maximaux peuvent ne pas produire des effets, autant que des stimuli minimaux, mais exprès codifiés, peuvent avoir des effets maximaux dans la biostructure.

Les zones d'élection pour l'application des excitations électriques et électromagnétiques en but thérapeutique sont les points électrodermiques actifs, les points d' acuponcture traditionnelle, les zones "trigger", les points moteurs Erb, les points Ryodoraku, Hirata, Weihe, Martinet, Voll, Dujardin, Walleix, Wetterwald, les points auriculaires et les dermatomes Head. Ces surfaces de projection cutanée des modifications biophysiques lesionnaires sont utilisées différemment dans les nombreuses techniques de stimulothérapie trans- et pércutanée.

Beaucoup de ces points et zones actives cutanées de corrélation avec des processus pathologiques sont caractérisés par les propriétés suivantes:
- conductivité intermédiaire entre metal et diélectrique
- conductivité unidirectionnelle
- la conductivité augmente proportionellement avec l' augmentation de la température

- potentiels électriques avec des valeurs élévées par rapport aux zones adjacentes indifférentes
- résistance électrique nettement diminuée et qui présente une variation cyclique
- niveau de stimulation similaire au courant d'ouverture des sémiconducteurs.

Ces propriétés biophysiques se modifient dans les maladies des organes dont la zone cutanée est corrélée et la disposition topographique est caractéristique pour l'organe affecté.

Les stimuli électriques et électromagnétiques appliqués aux niveaux des zones électrodermiques actives font appel aux structures neuro-réceptrices locales, en produisant des messages d'information sensorielle différentiés qui, transmis vers le cortex, sont soumis à l'induction réciproque et à l'inhibition. Ces messages causent l'apparition de certains potentiels evoqués, qui rétablissent, par la voie afférente, les zones de polarité anormale induites par les diverses affections au niveau tissulaire.

On peut affirmer alors que le résultats thérapeutiques obtenus actuellement par l'application des excitations électriques et électromagnétiques sont explicables par les effets suivants:
- bioélectroniques et biochimique, locaux et systèmiques
- de champ électromagnétique
- de résonce d'information
- mécanismes de contrôle au niveau médullaire et central
- l'action des neuro-médiateurs et des substances biologiquement actives

Les effets locaux et systèmiques: biostimulants, anti-inflammatoires, miorelaxants, anti-algiques, anesthésiques, neurostimulants, de réglement végétatif, de commande fonctionnelle des organes sont dépendents des zones d'application de l'excitant, du type, des paramètres et des modalités d'application.

Réferences bibliographiques: 1...9, 11, 12, 14, 17, 19, 20, 22, 23, 25, 27, 28, 30, 32...36, 39.

Stade actuel

Des diverses méthodes sont connues pour la stimulation électrique et photostimulation, appliquées en but thérapeutique (10, 13, 15, 21,26,29,31,38,40).

Les méthodes de stimulothérapie électrique sont des procédés qui utilisent l'énergie électrique en but thérapeutique. Elles font partie du domaine de l' Electrothérapie, qui fait l'object d'une grande partie de la Physiothérapie.

A1. La stimulothérapie électrique s'applique soit au niveau de certains points cutanés à localisation précise - trans ou pércutanée - par des techniques d' électroponcture ou électroacuponcture, soit par l' application diffuse des stimuli sur une surface plus grande de tégument: les techniques de stimulation électrique nerveuse trans-cutanée, soit par stimulation des cordons postérieurs modulaires, soit par des stimulations nerveuses centrales (région périapéductale, région périventriculaire, région thalamique, etc.).

L' électroponcture et l'électroacuponcture sont deux techniques qui s'appliquent aux niveaux des points d' acuponcture traditionnelle pour obtenir les deux types d' effets traditionnels: tonification-stimulation et dispersio-sédation. La neurostimulation transcutanée est utilisée pour obtenir une hypalgesie par l'application des stimuli sur la zone douloureuse.

La stimulation électrique nerveuse centrale est une méthode d'exception qui utilise les impulsions électriques pour modifier le seuil de perception douloureuse.

Les paramètres caractéristiques de tous les générateurs de courant électrique utilisés dans la stimulothérapie sont: le courant continu ou alternatid asymétrique, la forme d'impulsion (dans le cas du courant alternatif), l' intensité du courant, la fréquence de répetition des impulsions, le rythme de libération des stimuli. Le courant électrique ainsi généré s'applique soit au tégument, soit sur une autre zone d'élection par l' intermédiaire d'une paire d'électrodes, qui peuvent être: électrodes-aiguilles qui évitent l'ahaute impédance du tégument en le percant ou électrodes de contact, ce qui évite l'introduction des aiguilles dans le corps.

A2. Dans tous les procédés d'électrostimulothérapie connus l'identification des zones de stimulation s' effectue par une ou plusieures méthodes de mesure (8, 9, 15, 31, 32, 33).

Ils sont connus des méthodes et d'appareils pour l' identification des points actifs cutanés basés sur la proprieté de résistance électrique minimale de ceux-ci, en camparaison avec les zones adjacentes. Ces appareils fournissent une information d'orientation sur la distribution des valeurs résistives sur le tégument, en présentant un électrode mobile, une conductibilité de contact constante dans un régime qui élimine les tensions de polarisation. La résistance ohmique des points actifs ainsi mesurée est nettement baisée.

Avec ces appareils l'operateur interactionne avec les paramètres mesurés volontairement ou involontairement, en modifiant la pression de contact ou l'angle de contact peau-électrode. L'information ainsi obtenue est seulement d'orientation et ne permet pas la caractérisation du point de vue de la zone identifiée qui va être stimulée. Il y a des appareils pour l'identification des zones actives cutanées et pour établir le diagnostique électrodermal, en but d'application d'un traitement stimulothérapique (37). Les électrodes multiples de contact sont fixés sur le tégument et l'exploration s' effectue en courant de haute fréquence, qui, en combinaison avec les processus bioélectriques de basse fréquence au niveau de tégument, induisent dans le circuit un paramètre constant qui explore l'activité du derme et de l'épiderme et la capacité électrique de la membrane de base associée avec les charges électriques stockées d'une part et de l'autre de celle-là et pas de la couche cornée comme dans le premier set de méthodes. Cet appareil présente des avantages sur les appareils précédents en regard des interactions de contact et de la polarisation des électrodes, ainsi que le fait qu'il peut enregistrer simultanément plusieurs points actifs:

L'appareil présente les desavantages d'utiliser une proprieté passive du tégument, la résistance électrique, enregistrée dans un seul point et sans aucune corrélation avec l'état bioélectrique de la surface périproximale. Il est connu un appareil basé sur l'enregistrement de l' impedance électrique cutanée (16). Cet appareil met en evidence, par un emplacement spécial des électrodes multiples dans lesquels on introduit un courant électrique de 2 V à 13 Hz, les modifications de l' impedance cutanée, en fonction du temps d'exploration et des processus pathologiques des organes adjacents aux cadrans dans lesquels sont placés les électrodes. L' appareil est différent des précédents par la possibilité de contrôle partiel de la stimulo-thérapie appliquée et par la mise en evidence des capacités de bioréglage énergétique de la procédure utilisée.

Les modifications bioélectriques captées sont identifiées sur un surface de contact de grandes dimensions (diamètre env. 6 cm), donc diffuses et pas discrètes et le contrôle de la stimulo-thérapie s'effectue seulement au niveau d' orientation.

Il est connu un appareil utilisé dans les techniques de stimulo-thérapie et réglage bioénergétique qui utilise la combinaison des techniques de mesure de la conductivité électrique évoquée au niveau des points actifs cutanés avec l'association des petites doses de substances chimiques qui modifient différement la conductivité des zones actives en fonction des processus pathologiques des organes ou des systèmes dont ils sont corrélés (18). La méthode et l'appareil présentent des différences par rapport aux appareils et méthodes precédents. Pourtant la mfhode est insuffisamment fondée scientifiquement et expliquée du point de vue biophysique et ne peut offrir qu'un contrôle d'orientation pour les procédures de stimulo-thérapie.

On connait une méthode et un appareil qui combinent une technique d'enregistrement ponctuelle de la résistance électrique cutanée avec celle d'électrostimulation du point actif enregistré (41). L'appareil utilise des impulsions électriques de basse fréquence (8 - 10 Hz) avec une durée de 120 - 350 ms et une pause de 125 ms. L'appareil présente une echelle graduée qui correspond aux états normal jusqu'à l'irritation physiologique cumulative, inflammation, dégénération, etc. établie empiriquement. Le traitement consiste dans la libération, sur les points étudiés, d'une sèrie d'impulsions de courant électrique destinées à ramener sur l'echelle graduée la valeur mesurée en état normal physiologique. L'appareil présente les desavantages de mesurer et traiter un seul point cutané actif, d'une méthode empirique et pratiquement l'appareil ne présente aucune boucle de réaction entre le paramètre mesuré et les paramètres de l'excitant électrique libéré.

En conclusion, les méthode et les appareils qui utilisent le courant électrique en but stimulo-thérapeutique présentent les desavantages suivants:

1. l'application des paramètres de stimulation électrique n'est pas contrôlé, donc pas reproductible;

2. l'identification et la caractérisation des zones qui vont être électrostimulées s'effectue dans un seul point, sans préciser la variation des caractéristiques bioélectriques sur la zone périproximale;

2. les caractéristiques bioélectriques mesurées couramment par les appareils d'identification des zones actives sont des proprietés électroques passives du tégument (résistance et impedance électrique). L' exploration de ces proprietés passives présente des nombreux désavantages régardant les interactions de contact, la polarisation d'électrode, l'induction des perturbations bioélectriques dans la couche de base par l'injection du courant électrique, la quantification difficile des variations mesurées pour la faible reproductibilité du phénomène mesuré.

4. il n'y a aucune rélation établie entre les proprietés bioélectriques des zones soumises à la stimulation électrique et les paramètres d'application des stimuli.

5. les caractéristiques constructives des générateurs d' impulsions électriques connus ne permettent pas la combinaison et l'application en proportions variables des stimuli électriques.

6. les appareils et les méthodes de stimulation électrique ne permettent pas d'établir automatiquement la polarité des électrodes, paramètre important de la stimulation électrique, duquel dépend l'obtention des effets thérapeutiques qualitatifs, en fonction des caractéristiques bioélectriques des zones sélectionnées pour la stimulation.

7. les appareils et les méthodes de stimulo-thérapie électrique connus ne permettent pas d'obtenir un effet thérapeutique escompté par par individualisation des paramètres d'application de l'excitant électrique en fonction des données cliniques, diagnostiques et de mesure des zones d'application des stimuli, variables individuelles du malade traité.

8. les appareils et les méthodes connus ne permettent pas la conduite du traitement par "biofeedback" entre le signal électrique appliqué, les variables de l' électrodynamique de la surface cutanée d'application et l'effet thérapeutique escompté.

B1. On connait des divers types de lasers et des méthodes d'application de la radiation électromagnétique cohérente en but thérapeutique: laser He-Ne avec pompage optique et puissance de 1 à 15 mW; lasers au bioxide de carbone défocalisés avec pompage électrique et puissance de moins de 50 mW; lasers à sémiconducteurs avec pompage électrique et puissance de 1 à 15 mW; lasers Nd-Yag ou Nd-Ylf avec pompage électrique et puissance de 5 à 20 mW. Ces types de lasers utilisés dans la stimulothérapie ont des effets biostimulants, antialgiques, antioedèmateux, etc. et agissent probablement par la modification des potentiels de membrane, la libération des neuromédiateurs chimiques et par effet du champ électromagnétique.

On connait des méthodes d'application de radiation électromagnétique cohérente: point par point - laserstimulothérapie, ou l'identification et la caractérisation des points actifs s'effectue conformément aux méthodes decrites antérieurement; par balayage automatique de la région anatomique qui présente une modification pathologique - laserscanning.

B2. Les désavantages de ces méthodes sont les mêmes que ceux énumérés au paragraphe concernant l' èlectrostimulothérapie. En plus l'application de la laserstimulation se fait point par point, sans la modification des paramètres: fréquence, puissance, modulation des impulsions; donc il n'y a pas la possibilité de produire des pheñomènes d'interférence entre deux zones excitées et de résonance.

C. Il n'y a pas des méthodes et des appareils pour l' application simultanée et contrôlée dans un boucle de réaction de type "bio-feedback" du champ électrique et électromagnétique cohérent, dans le but d'obtenir des effets thérapeutiques différentiés par des cpmbinaisons dans diverses proportions des signaux électriques et optiques appliqués au niveau de la surface cutanée, dans des protocoles différents de ceux utilisés par la médecine traditionnelle ou complémentaire.

Plus précisement la présente invention se réfère à un procéde pour le traitement d'information en champ électrique et électromagnétique caractérisé en ce qu'on utilise l'application simultanée et contrôlée du champ électrique de basse et infra-basse fréquence et de la radiation électromagnétique cohérente emise par deux sources laser de faible puissance et paramètres d' excitation différents; l'établissement de ces paramètres et le contrôle de ces applications s'effectuant dans la séquence suivante:

- on identifie et on effectue une sélection successive des zones électrodermiques proposées initialement pour la stimulation en déterminant un paramètre dépendant du potentiel et da la résistance électrique cutanée, mesurée par une méthode atypique, en utilisant une chaîne de mensuration avec une impédance d'entrée comparable à celle du tégument, defini comme coefficient de transfert électrocinétique et réprésentant la mesure de l'activité électrocinétique, évaluée sur une échelle arbitraire; la sélection des dites zones est faite par rapport à un seuil individuel établi dans le moment de l'exploration par rapport aux variations de l'électrocinétique locale; suite à la sélection on obtient un set de zones électrodermiques actives plus petit ou égal au set initial recommandé;

- on propose un set de paramètres d'application du champ électrique: le montage spatial des paires d'électrodes, le train et les modalités de génération des impulsions, la forme des impulsions, le montage interférentiel résultant de la combination du train et des modalités de génération des impulsions avec le montage spatial des paires d'electrodes; ce set est developpé d'un set initialement établi par des méthodes expérimentales et statistiques;

- dans le zones sélectionnées dans le montage spatial recommandé on place successivement les électrodes matriciels à double fonction slternative, sensorielle et excitante et on établit les coefficients de transfert électrocinétique pour chaque surface élémentaire;

- on détermine l'activité électrocinétique de surface des zones sélectionnées en calculant le gradient électrocinétique local , réprésentant la distribution des coefficients de transfert électrocinétique pour la surface cutanée ainsi mesurée et le gradient électrocinétique global qui représent la mesure de l' activité électrocinétique distribuée au niveau des zones sélectionnées;

- on établit automatiquement la polarité des électrodes matriciels pour les zones sélectionnées, séparement pour les zones destinées pour l'excitation en champ électrique et celles pour l'excitation en radiation cohérente: on cherche la zone caractérisée par un gradient électrocinétique maximal en attribuant la polarité negative et on attribue la polarité positive à la zone paire;

6

- on propose deux sets différents des paramètres d' application de la radiation électromagnétique cohérente: la longeur d'onde, la puissance, la fréquence des trains d'impulsions et les modalités d' application, variables en fonction des données expérimentales et statistiques; on attribue au premier set les zones de polarité negative, en écartant les électrodes matriciels correspondants.

- on applique le champ électrique pour un intervalle de temps préetabli et la radiation cohérente pour un intervalle de temps plus petit ou égal au premier; suite à l'application simultanée du champ électrique et électromagnétique aux paramètres individualisés et en fonction de l'activité électrodynamique des zones soumise à la stmulation conformément à la méthode, se produisent au niveau des zones d'application des effets bioélectroniques, biochimique, de champ électromagnétique et de résonance d'information; l' amplification de ces effets locaux conduit à l' apparition des effets systèmiques par l'action des neuromédiateurs, des substances biologoques actives, des hormones, des mécanismes de contrôle au niveau médullaire et central etc.; l'application des champs électriques et électromagnetiques conduit aux effets locaux et systèmiques différents, en fonction de la modulation de ceux-ci; après l'excitation on mesure un nouveau set des coefficients de transfert électrocinétique, on clalcule un nouvea set des gradients électrocinétiques locaux et on calcule l' indice global de l'effet de l'excitation sur l' activité électrocinétique;

- les dites données: les variables individuelles, les sets des zones et les paramètres d'application initialement recommandés, les sets des zones sélectionnées, l'evolution des polarités, des paramètres d'application des excitants et l'indice global de l'effet de l'excitation sur l'activité électrocinétique, qui représente la mesure et l' evolution de la résonance d'information du traitement sont analysées par des méthodes statistiques et euristiques; suite à l'analyse on peut déterminer des sets optimaux initiaux d'excitation électrique et électromagnétique cohérente.

Le procéde de traitement d'information en champ électrique et électromagnétique est caractérisé, en but de l'obtention des effets thérapeutiques différenciés et de l'accroissement progressif de l'efficacité de ceux-ci, par le fait qu'on utilise l'application simultanée et contrôlée du champ électrique de basse et infra-basse fréquence et de la radiation électromagnétique cohérente emise de deux sources laser de faible puissance et paramètres d'excitation différents. l'etablissement de ces paramètres et le contrôle de ces applications en but thérapeutique s' effectue dans la séquence suivante:

- on enregistre les données individuelles du patient: âge, sexe, état général et les données diagnostiques établies cliniquement et par des mowens paracliniques d'investigation: type, localisation anatomique, étiologie et activité du processus pathologique et le symptome principal de manifestation de la maladie.

- on établit, initialement, un ou plusieurs effets thérapeutiques escomptés d'une série d'effets thérapeutiques possible à obtenir; au cours du traitement on estime l'effet thérapeutique obtenu suite à la sénce thérapeutique précédente, en attribuant un coefficient d'accomplissement sur une échelle arbitraire, on estime l'effet thérapeutique global comme une somme ponderée de coefficients d' accomplissement correspondants aux effets thérapeutiques selectionnés.

- on recommande, initialement, un set variable de zones paires pour la stmulation en champ électrique et un set variable pour la stimulation avec radiation cohérente; l'emplacement anatomo-topographique de ces zones et les sets recommandés sont établis experimentalement et par moyens statistiques en fonction du diagnostic et sont dépendents des effets thérapeutiques escomptés selectionnés.

- on identifie et on effectue une sélection successive des zones électrodermiques recommandées initialement proposées pour la stimulation en but thérapeutique en déterminant un paramètre dépendent du potentiel et de la résistance électrique cutanée, mesurée par une méthode atypique, en utilisant une chaîne de mensuration avec une impédance d'entrée comparable à celle du tégument, défini comme coefficient de transfert électrocinétique et représentsnt la mesure de l' activité électrocinétique, évaluée sur une échelle arbitraire; la sélection des dites zones est faite par rapport à un seuil individuel établi dans le moment de l'exploration par rapport aux variations de l' électrocinétique locale; suite à la sélection on obtient un set de zones électrodermiques actives plus petit ou égal au set initial recommandé.

- on recommande un set de paramètres d'application du champ électrique: le montage spatial des paires d' électrodes, le train et la modalité de génération des impulsions, la forme des impulsions, le montage interferéntiel résultant de la combinaison du train et de la modalité de génération des impulsions avec le montage spatial des paires d'électrodes; ce set est développé d'un set initialement établi par des méthodes expérimentales et statistiques en fonction des données d'état, des données diagnostiques du patient et des effets escomptés pour le set variable des zones électrodermiques recommandées initialement et actualisé dans les conditions particuliéres résultant suite à la sélection des zones électrodermiques actives.

- dans les zones sélectionnées conformement à la méthode exposée, dans le montage spatial recommandé on place successivement les électrodes matriciels à double fonction alternative, sensorielle et excitante et on établi les coefficients de transfert électrocinétique pour chaque surface élementaire.
- on détermine l'activité électrocinétique de surface des zones sélectionnées en but thérapeutique, en calculant le gradient électrocinétique local, représentant la distribution des coefficients de transfert électrocinétique pour la surface cutanée ainsi mesurée et le gradient électrocinétique global qui représente la mesure de l'activité électrocinétique distribuée au niveau des zones sélectionnées.
- on établi automatiquement la polarité des électrodes matriciels pour les zones sélectionnées, séparement pour les zones destinées pour l'excitation en champ électrique et celles pour l'excitation en radiation cohérente: on cherche la zone caractérisée par un gradient étectrocinétique local maximal en attribuant la polarité negative et on attribue la polarité positive à la zone paire.
- on recommande deux sets différents des paramètres d' application de la radiation électromagnétique cohérente: la longeur d'onde, la puissance, la fréquence des trains d'impulsions et les modalités d' application, variables en fonction des donnèes d'état, du diagnostic du malade et des effets escomptés et établis par des méthodes experimentales et statistiques; on attribue au premier set les zones de polarité negative et au deuxième set les zones de polarité positive, en écartant les électrodes matriciels correspondants.
- on applique le champ électrique pour un intervalle de temps préétabli et la radiation cohérente pour un intervalle de temps plus petit ou égal au premier; suite à l'application simultanée du champ électrique et électromagnétique aux paramètres individualisés, en fonction du patient, du diagnostic et de l#activité électrodynamique des zones soumises à la stimulation conformément à la méthode, se produisent au niveau des zones d'application des effets bioélectroniques, biochimiques, de champ électromagnétique et de résonance d'information; l'amplification de ces effets locaux conduit à L#apparition des effets systèmiques par l'action des neuromédiateurs, des substances biologiques actives, des hormones, des mécanismes de contrôle à niveau médullaire et central, etc.; l' application des champs électriques et électromagnétiques conduit à des effets locaux et systèmiques différents et alors à des effets thérapeutiques différenciés, en fonction de la modulation de ceux-ci.
- après l'excitation on mesure un nouveau set des coefficients de transfert électrocinétique, on calcule un nouveau set des gradients étectrocinétiques locaux et on calcule l'indice global de l'effet de l' excitation sur t'activité étectrocinétique.
- on defini comme séance thérapeutique la séquence présentée; on definit comme intervalle de décision thérapeutique un nombre préétabli de séances thérapeutiques; l'effet thérapeutique cumulé obtenu par l'addition des effets thérapeutiques globaux sur l' intervalle de décision thérapeutique est encadré dans un intervalle d'appréciation parmi les quatre possibles: effet thérapeutique nul (I1), effet thérapeutique moyen (I2), effet thérapeutique bon (I3) et effet thérapeutique très bon (I4).
- en fonction de l'intervalle d'appréciation où l'effet thérapeutique cumulé est encadré, une décision est prise conformément à un algorithme où les décisions possibles sont:
  - la décision STOP = arrête l'application du traitement dans le situations suivantes:
    1. après un nombre maximal recommandé de séances thérapeutiques dans lesquelles le même set de paramètres a été appliqué et l'effet thérapeutique cumulé a été encadré dans les intervalles I3, I4;
    2. après un nombre maximal recommandé de séances thérapeutiques dans lesquelles des sets différents de paramètres ont été appliqués après la décision REEVALUATION et l'effet thérapeutique cumulé a été encadré dans les intervalles I2, I3, I4;
    3. après deux intervalles de décision thérapeutique consécutifs entre lesquels la décision REEVALUATION a été prise et l'effet thérapeutique cumulé a été encadré dans l'intervalle I1.
  - la décision CONTINUE = cause l'application du set des paramètres d'excitation établis, sans aucune modification jusqu'à la décision suivante.
  - la décision REEVALUATION = cause la modification du set des paramètres d' excitation établis précédemment, en établissant un nouveau set d'effets thérapeutiques escomptés
  - la décision STOP/MEMORISE = cause la mémorisation des données déterminées pendant le traitement, seulement pour les patients encadrés dans l'intervalle I4 à la fin du traitement.
- les dites données, les variables individuelles, les effets thérapeutiques escomptés, les sets de zones et les paramètres d'application initialement recommandés, les sets de zones selectionnées, l'evolution des polarités, des paraMétres d'application des excitants et de l'indice global de l'effet de l'excitation sur l' activité électrocinétique, qui represente la mesure de la résonance d'information du traitement en comparaison avec l'évolution clinique, estimé par l'evolution des coefficients de l'effet thérapeutique global et cumulé, sont analysés par des méthodes statistiques et on établit des groupes de données pour patients encadrés dans les mêmes classe diagnostiques et pour lesquels on a appliqué des sets différents des paramètres d' excitation; suite à l'analyse statistique on peut déterminer des sets optimals initials d'excitation électrique et électromagnétique cohérente en but thérapeutique.

Le dispositif pour la mise en oeuvre du procédé de l' invention est caractérisé par le fait qu'on prevoit une interface d'information sensorielle excitatrice, dans le but d'obtenir zn échange d'information/énergie d'une part et de l'autre d'une surface de séparation et la génération du phénomène de résonance d'information dans une biostructure, utilisant un set d'électrodes matriciels à double fonction alternative, de mesure et d' excitation en champ électrique, interconnecté avec un bloc d'analyse des données d'entrée et un bloc de commande des signaux de sortie; l'électrode matriciel est une surface de mesure/stimulation sur laquelle sont disposés un nombre d'électrodes de contact.

Le système de traitement d'information en champ électrique et étectromagnétique est caractérisé par le fait que, dans le but d'appliquer la méthode décrite ci-dessus, il est constitué d'un système multiprocesseur et bati autour d'une architecture d'ordinateur personnel (OP) avec des acessoires standard (clavier, moniteur couleur, disque rigide, disque flexible, imprimante) et muni d'une interface de communication avec les contrôleurs intelligents (CSE), (CSL); les contrôleurs CSE sont specialisés pour la stimulation électrique (champ électrique de basse et infra-basse fréquence) et aussi pour l'acquisition des données par l'intermédiaire des électrodes matriciels à double fonction, sensorielle et excitante, alternative; le contrôleur CSL est specialisé pour la stimulation en champ électromagnétique par radiation cohérente, en utilisant dans ce but des lasers de faible puissance et doté d'un crayon-détecteur connecté avec une chaîne d'acquisition pour les mensurations dans la phase de sélection des zones cutanées.

## 2. LE BUT

L'obtention des effets thérapeutiques différentiés et l'accroissement progressif de l'efficacité de ceux-ci.

## 3. LES MOYENS

Le système BIOTEN dépasse les désavantages des appareils existants en utilisant l'application simultanée du champ électrique et du champ électromagnétique avec des paramètres contrôlés en fonction des données cliniques et diagnostiques individuelles du malade, des effets thérapeutiques escomptés et des caractéristiques électrodynamiques enrégistrées au niveau des surfaces discrètes de l'enveloppe cutanée, premièrement recommandées, ensuite identifiées et sélectionnées.

## 4. LA DESCRIPTION

Pour obtenir des effets thérapeutiques différentiés et augmenter progressivement l'efficacité de ceux-ci, on utilise l'application simultanée et contrôlée da champ électrique de basse et infra-basse fréquence et de la radiation électromagnétique cohérente emise par deux sources laser de faible puissance et paramètres d' excitation différents. L'application de ces stimuli s' effectue conformément à la methode suivannte.

a.- On enrégistre les données suivantes, variables individuelles du patient:
a.1.- les paramètres d'état du malade:
a.1.1.- l'âge
a.1.2.- le sexe
a.1.3.- l'état général actuel du malede, apprecié sur l'échelle de Karnovski (0...100 points)
a.2.- certaines données diagnostiques, établie cliniquement et par moyens paracliniques d' investigation:
a.2.1.- le type du processus pathologique
a.2.2.- la localisation anatomique du processus pathologique
a.2.3.- l'étiologie du processus pathologique
a.2.4.- l'activité du processus pathologique (aigu, chronique)
a.2.5.- le symptôme principal de manifestation de la maladie
b.- Initialement on établit, en particularisant en fonction du patient, un ou plusieurs effets thérapeutiques escomptés, en fonction de la maladie et des caractéristiques individuelles de manifestation au malade ainsi traité. Les effets sont extraits d'une sèrie d'effets thérapeutiques possibles à obtenir. Examples:
b.1.- effet antialgique
b.2.- effet antiinflammatoire

9

b.3.- effet vasodilatateur

b.4.- effet décontractant musculaire

b.5.- effet neurostimulant etc.

Au cours du traitement on estime l'effet thérapeutique obtenu suite à la séance thérapeutique précédente. L'estimation se fait pour chaque effet thérapeutique escompté, en attribuant des coefficients d'accomplissement CAi sur une échelle arbitraire. On estime l'effet thérapeutique global ETG comme une somme pondérée des coefficients CA correspondants aux "n" effets thérapeutiques escomptés sélectionnés.

$$ETG = \frac{1}{n} \sum_{i=1}^{n} Ki \times CAi$$

où Ki = coefficient poids déterminé par des méthodes statistiques

c.- On recommande, initialement, un set variable x1 de zones paire pour la simulation en champ électrique et un set variable x2 des zone paire pour la stimulation en radiation cohérente. L'emplacement anatomotopographique de ces zones et les sets recommandés sont établis expérimentalement et par méthodes statistiques en fonction du diagnostic et sont dépendants des effets thérapeutiques escomptés sélectionnés

x1, x2 (a.2.1; a.2.2; a.2.3; a.2.4; a.2.5; b)

Ces zones recommandées pour la stimulation conformement à la méthode exposée sont des zones électrodermique actives de l'enveloppe cutanée. Elles présentent une résistance électrique basse, un potentiel et un capacité électrique élévés, en comparaison avec les zones adjacentes, indifférentes du point de vue électrodynamique. Les proprietés biophysiques distinctes se modifient dans les maladies des organes dont elles sont corrélées, déterminant à leur surface des transferts de charges électriques, des chargements triboélectriques, des variations importantes de la température.

d.- On identifie et on effectue une sélection successive des zones électrodermiques proposées pour la stimulation en but thérapeutique. Ces zones présentent une résistance électrique diminuée et un potentiel électrique élevé par rapport aux zones périproximales.

Dans le système BIOTEN il est nécessaire de détrminer un paramètre qui caractérise aussi la surface de transfert d'énergie entre l'organisme et le milieu que le degré d'activité de ce transfert. Dans ces conditions on a choisi une méthode atypique de mesure du potentiel électrique, en utilisant une chaîne de mensuration avec une impédance d'entrée comparable a celle du tégument. Ainsi on peut déterminer un paramètre dépendant du potentiel et de la résistance électrique en même temps. Ce paramètre est dénommé coefficient de transfert électrocinétique CTE et réprésente la mesure de l'activité électrocinétique, evaluée sur une échelle arbitraire. La sélection des zones est faite par rapport à un seuil individuel établi CTEs (seuil) dans le moment de l'exploration par rapport aux variations de l' électrocinétique locale.

On obtient ainsi un nombre M des zones électrodermiques actives avec:

M = Mx1 + Mx2

avec:

Mx1 ≤ x1

Mx2 ≤ x2

e.- On recommande un set (E) de paramètres d'application du champ électrique:

- le montage spatial des paires d'électrodes
- le train et les modalités de génération des impulsions
- la forme des impulsions
- le montage par interférence résultant de la combinaison du train et des modalités de génération des impulsions avec le montage spatial des paires d'électrodes

Ce set est développé d'un set initialement établi par des méthodes expérimentales et statistiques en fonction des conditions (a.1.2; b), pour le set variable des zones x1, dans les conditions particulières résultant suite à la sélection des zones électrodermiques actives (d).

f.- Dans les zones sélectionnées conformément à la méthode exposée (d), dans le montage spatial recommandé (e) on place successivememt les électrodes matriciels.

L'électrode matriciel est une surface de mesure/stimulation S sur laquelle sont disposés un nombre N d'électrodes de contact avec une double fonction, alternative, de mesure/excitation en champ électrique:

$$S = \sum_{i=1}^{N} p_i$$

où pi est une surface élementaire de mesure/excitation électrique

Dans les zones sélectionnées on établit, par la méthode de mensuration (d), les coefficients de transfert électrocinétique CTEij pour chaque surface élementaire avec:

$i = 1....N \qquad j = 1....M$

L'électrode matriciel, comme limite de séparation entre le système organique et un système composé des sous-ensembles de mensuration et d'excitation connectés avec un bloc d'analyse des données d'entrée et un bloc de commande des signaux de sortie, peut être defini comme une interface d'information sensorielle-excitatrice (IISE).

IISE permet l'échange de l'information-énergie d'une part et de l'autre de la surface de séparation S et la génération du phénomène de résonancè d' information.

g.- Pour détrminer l'activité électrocinétique de surface des zones sélectionnées en but thérapeutique, on calcule le gradient électrocinétique local GELj, qui représente la distribution du coefficient CTEij sur la surface cutanée Sj (pour $j = 1....M$):

$$GEL_j = \frac{\overline{CTE_j}}{CTE_s} \ast \frac{M + \sum_{i=1}^{M} sgn\ (CTE_{ij} - CTE_j)}{2}$$

Le gradient électrocinétique local GEG représente une mesure de l'activité électrocinétique distribuée au niveau des zones sélectionnées

$$GEG = \overline{GEL} \ast \frac{M + \sum_{i=1}^{M} sgn\ (GEL_j - \overline{GEL})}{2 \ast M}$$

h.- On établit automatiquement la polarité des électrodes matriciels pour les M zones sélectionnées, séparément pour le sets Mx1 et Mx2, ainsi:

1.- on cerche la zone caractérisée par GELmax et on lui attribue la polarité negative;

2.- on attribue la polarité positive à la zone paire;

3.- on élimine la paire ainsi trouvée et on recommence les pas 1. et 2. pour toutes les zones recommandées restantes.

i.- On recommande deux sets différents de paramètres d' application de la radiation électromagnétique cohérente, variables en fonction de (a.1, a.2, b) et établis par des méthodes expérimentales et statistiques. Les paramètres d'interêt des deux sets L1 et L2 sont: la longueur d'onde, la puissance, la fréquence des trains d'impulsions et les modalités d'application.

Suite à la détermination des polarités (h), on attribue le set L1 aux zones de polaritè negative et le set L2 aux zones de polarité positive, en écartant les électrodes matriciels correspondants.

j.- On applique le champ électrique pour un intervalle de temps TEmax et la radiation électromagnétique cohérente pour un intervalle de temps TLmax (TL max < < TE max).

Suite à l'application simultanée du champ électrique et électromagnétique aux paramètres individualisés, en fonction du patient, du diagnostic et de l' activité électrodynamique des zones soumises à la stimulation conformément à la méthode, se produisent au niveau des zones d'application des effets bioélectroniques, biochimiques, de champ électromagnétique et de résonance d'information. L' amplification de ces effets locaux conduit à l' apparition des effets systèmiques par l'action des neuromédiateurs, des substances biologiques actives, des hormones, des mécanismes de contrôle au niveau médullaire et central etc..

L'application des champs électrique et électromagnétique cinduit à effets locaux et systèmiques différents et alors à effets thérapeutiques différentiés, en fonction de la modulation de ceux-ci.

k.- Après L'excitation on mesure encore une fois les coefficients de transfert électrocinétique, dénommés pCTE, d'après la méthode decrite (f) et on calcule les gradients pGEL, pGEG d'après la méthode (g). Avec ces résultats on calcule l'indice global de l' effet de l'excitation sur l'activité électrocinétique IG:

$$IG = pGEG - GEG$$

l.- On definit les elements suivants:

1.- séance thérapeutique ST = les pas de la méthode (b)....(k)

2.- intervalle de décision thérapeutique IDT = un nombre V de séances thérapeutiques ST.

Avec les elements ainsi definis on peut évaluer l' effet thérapeutique cumulé ETC:

$$ETC = \frac{1}{V} * \left( \sum_{i=1}^{V} ETG_i + \Delta i \right) \quad \Delta i : \begin{cases} 1 ... ETG_{i+1} \geqslant ETG_i \\ 0 ... ETG_{i+1} < ETG_i \end{cases}$$

L'effet thérapeutique cumulé ainsi déterminé est encadré dans un intervalle d'appréciation parmi 4 possibles:

l1.- effet thérapeutique nul

l2.- effet thérapeutique moyen

l3.- effet thérapeutique bon

l4.- effet thérapeutique très bon

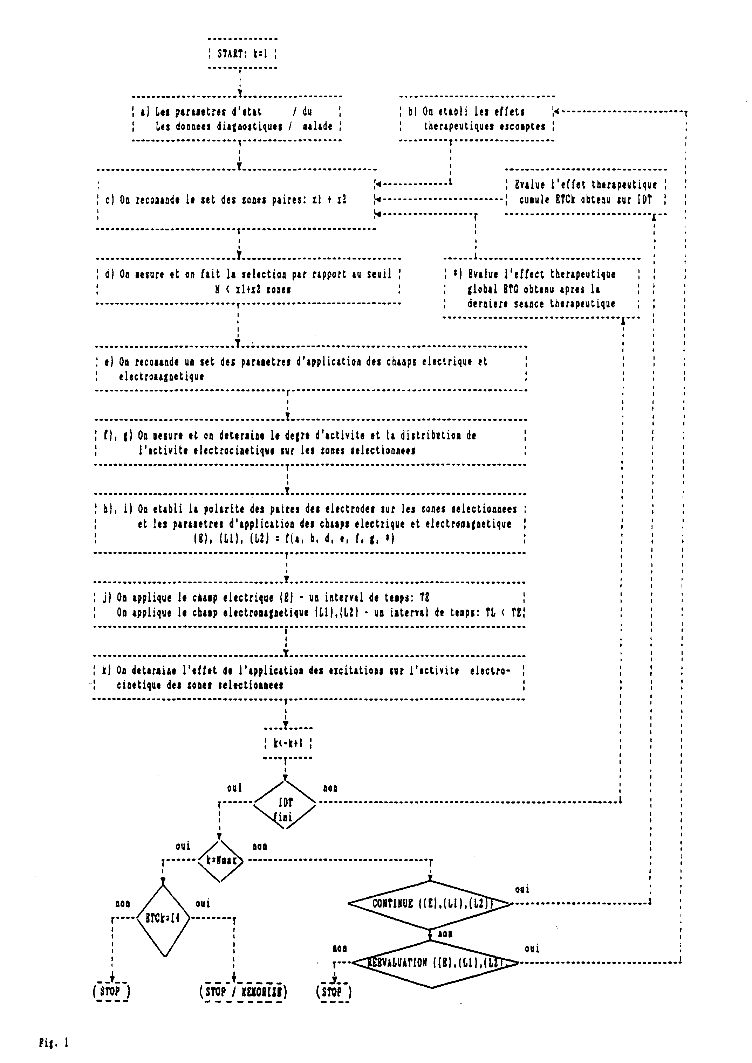

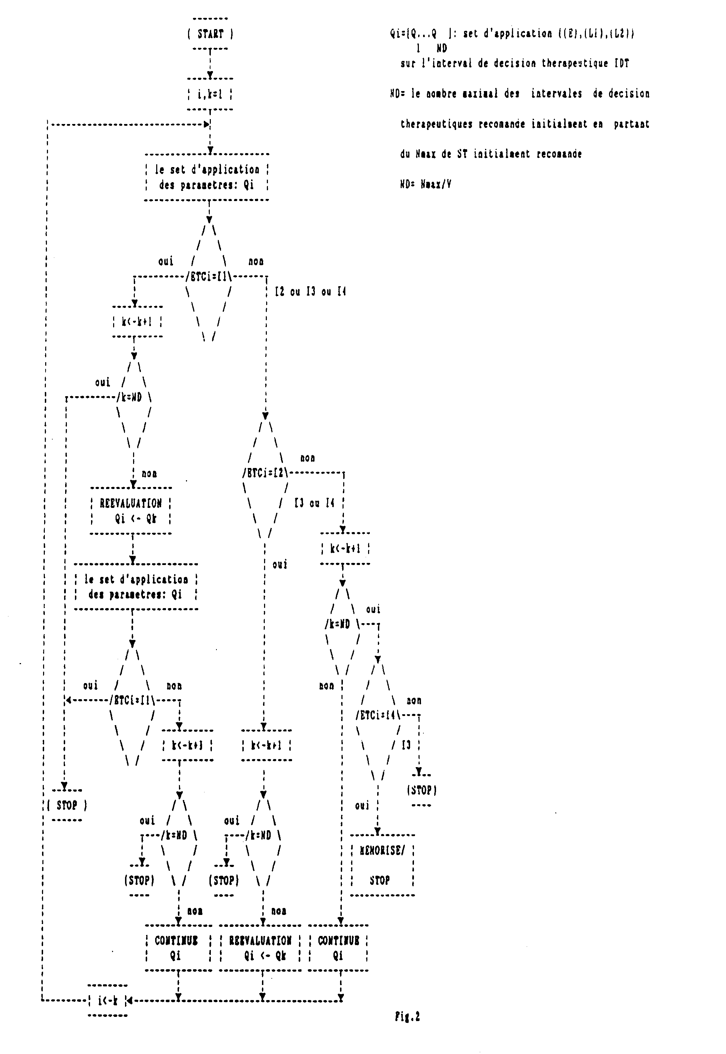

m.- En fonction de l'intervalle d'appréciation où l' effet thérapeutique est encadré, une décision est prise conformément à l' algorithme présenté dans la fig. 2:

Les significations des décisions sont:

1.- la décision STOP: arrête l'application du traitement dans les situations suivantes:

- après un nombre Nmax recommandé des ST dans lesquelles le même set des paramètres (E), (L1, L2) a été appliqué et ETC a été encadré dans les intervalles l3, l4.
- après un nombre Nmax recommandé des ST dans lesquelles des sets différents des paramètres (E), (L1, L2) ont été appliqués après la décision REEVALUATION et ETC a été encadré dans les intervalles l2, l3, l4.
- après deux IDT consecutifs, entre lesquels la décision REEVALUATION a été prise et ETC a été encadré dans l'intervalle l1.

2.- la décision CONTINUE cause l'application du set des paramètres d'excitation (E), (L1, L2) établis sans aucune modification jusqu'à la décision suivante.

3.- la décision REEVALUATION cause la modification du set des paramètres d'excitation (E), (L1, L2) établis précédemment, en établissant un nouveau set d'effets thérapeutiques escomptés.

4.- la décision STOP/MEMORISE cause la mémorisation des données déterminées pendant le traitement, seulement pour les patients encadrés dans l' intervalle l4 à la fin du traitement. Les dites données sont analysées par des méthodes statistiques et on établit des groupes de données pour patients encadrés dans les mêmes classes diagnostiques et pour lesquels on a appliqué des sets différents des paramètres d'excitation (E), (L1, L2). Les groupes de données sont: les variables individuelles, les effets thérapeutiques escomptés, les sets de zones et de paramètres d'application initialement recommandés, les sets de zones selectionnées, l' evolution des polarités, des paramètres d' application et de l'indice global de l'effet de l'excitation sur l'activité électrocinétique (IG), qui représente la mesure de la résonance d' information du traitement en comparaison avec l' évolution clinique, estimé par l'evolution des coefficients ETG et ETC. Suite à l'analyse statistique on peut déterminer des sets optimals initials (E), (L1, L2).

## 5. SCHEMA BLOC

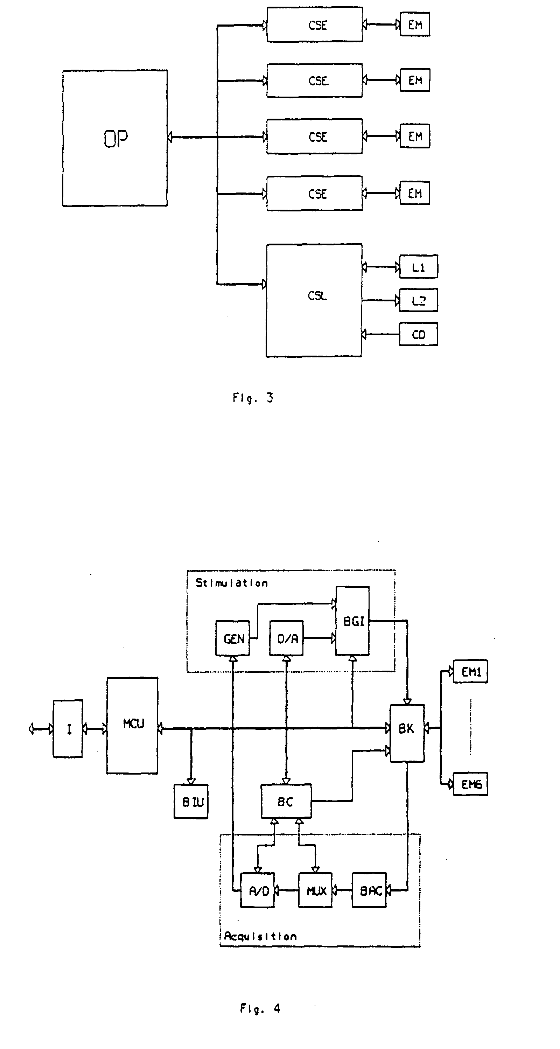

Du point de vue constructif le système BIOTEN est un système multiprocesseur, presenté dans le schéma de la fig. 3.

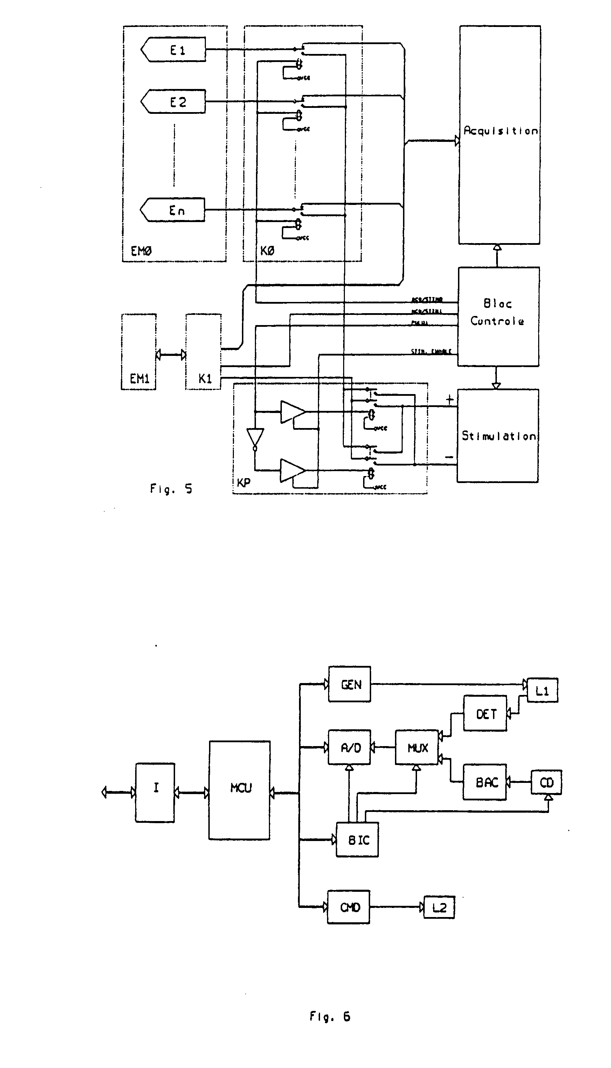

Il est bâti autour d'une architecture d'ordinateur personnel (OP) avec des accessoires standard (clavier, moniteur couleur, disque rigide, disque flexible, imprimante) et muni d'une interface de communication avec les contrôleurs intellegents (CSE), (CSL). Le logiciel qui tourne sur cet ordinateur consiste des deux bases de données et de connaissances, du système expert pour la sélection des zones cutanées d'interêt et pour l' élaboration des paramètres d'excitation en but thérapeutique, des modules de calcul statistique, des modules graphiques, des interfaces "utilisateur" et de communication avec les autres contrôleurs intelligents du système. Les autres ressources de calcul(acquisition, commande et contrôle des paramètres d'excitation etc.) sont distribuées parmi les contrôleurs (CSE), (CSL). Les contrôleurs (CSE) sont specialisés pour la stimulation électrique (champ électrique de basse et infra-basse fréquence) et aussi pour l'acquisition des données. L'interface de mesure et d'excitation se fait par l'intermédiaire des électrodes matriciels (EM) à double fonction, sensorielle et excitatrice, alternative. Le contrôleur (CSL) est spécialisé pour la stimulation en champ électromagnétique par radiation cohérente, en utilisant dans ce but les lasers (L1) et (L2). Pour les mensurations dans la phase de sélection des zones cutanées ce contrôleur est doté d'une chaîne d' acquisition et est muni d'un crayon-détecteur (CD). Le contrôleur (CSE) a le schéma bloc présenté dans la fig. 4.

Le microcontrôleur (MCU) en consiste le coeur, surveillant le fonctionnement des chaînes d'acquisition et de stimulation et des autres circuits. La communication avec le processeur "master" du système se fait par l'intermédiaire du circuit d'interface (I). Le bloc d'interface avec l'utilisateur (BIU) est responsable de la communication avec l'operateur. Le microcontrôleur (MCU) comporte aussi des circuits "timer" pour la réalisation d'un horologe de temps réel:

Le générateur programmable (GEN) produit des impulsions de commande à fréquence, durée et modalité de travail programmable. Le signal résultant et le signal provenant du convertisseur digital-analogique (D/A) commandent le bloc générateur d'impulsions (BGI), qui forme les impulsions de stimulation en champ électrique de fréquence comprise dans le domaine 1....1000 Hz. Ces impulsions sont distribuées par l'intermédiaire du bloc commutateur (BK) aux 3 paires d'électrodes matriciels à fonction excitatrice (EM).

Le bloc (BK) permet également de connecter un des électrodes matriciels à l'entrée de la chaîne d' acquisition des données. Cette chaîne est formée d'un bloc d'amplification et conditionnement des signaux (BAC) où les signaux prélevés par les électrodes matriciels à fonction sensorielle sont convertis et amplifiés, du circuit multiplexeur (MUX) et du convertisseur analogique-digital (A/D), le tout commandé par le bloc de contrôle (BC).

Une description plus detaillée d'une paire d'électrodes matriciels (EM) et du bloc commutateur (BK) est donnée dans la fig. 5.

Un électrode matriciel (EMO) est constitué d'un support élastique et isolant du point de vue électrique sur lequel un certain nombre d'électrodes de contact (E1).... (En) est placé.

Le bloc de commutateurs (KO) commande une des deux fonctions des électrodes: sensorielle ou excitante (ACQ/STIMO), connectant les élements de contact soit à l' entrée du bloc d'acquisition soit à la sortie du bloc de stimulation.

Chaque électrode de contact est connecté individuellement à une chaîne d'acquisition du potentiel électrique cutané et au moment d'application des excitations électriques tous les électrodes de contact sont unis et connectés sur une ligne positive ou negative de la chaîne de stimulation, en fonction des recommandations faites par le système. La surface de contact ainsi constituée correspond à la géométrie de l'électrode matriciel appliqué. A cause de cela le nombre et la géométrie des électrode de contact sur l'électrode matriciel dépend de la surface et de l'activité électrocinétique de la zone explorée et sélectionnée pour la stimulation.

Le bloc (KP) de commutateurs permet la sélection de la polarité appliquée sur les electrodes paires (EMO) et (EM1). Les commutateurs (KO), (K1) et (KP) font partie du bloc commutateur (BK) mentionné plus haut.

Cette configuration assure une précision spatiale parfaite des mensurations avant et après la stimulation.

Le contrôleur (CSL) a le schéma bloc présenté dans la fig. 6.

13

Le fonctionnement est contrôlé par le microcontrôleur (MCU), qui posséde aussi une horloge de temps réel et qui est subordonné au processeur "master" du système par l' intermédiaire du circuit interface de communication (I). Les lasers utilisés sont des lasers de faible puissance, dans le domaine 2...10 mW.

La commande du laser (L2) est réalisée par le bloc (CMD). Le contrôle du fonctionnement du laser (L1) est assuré par le générateur programmable (GEN), qui permet la commande de la puissance de l'emission et des modalités d'application des impulsions et par le circuit détecteur (DET) pour la monitorisation du coefficient d'absorption de la radiation, mesuré par la méthode de réflexion. Pour les mensurations préliminaires des zones recommandées par le système le contrôleur (CSL) est doté d'un crayon-détecteur (CD) couplé à une chaîne d' acquisition similaire à celles utilisées par les électrodes matriciels, bloc pour l'amplification et le conditionnement du signal (BAC), multiplexeur pour sélectionner les signaux mesurés et convertisseur analogique-digital (A/D).

Le bloc d'interface et de contrôle (BIC) assure l' interface avec l'operateur et les signaux de commande nécessaires.

## 6. MODE D'EMPLOI

Le mode d'emploi du système BIOTEN est le suivant:

a) - Au début d'un traitement l'operateur enrégistre les données individuelles concernant le patient: âge, sexe, état général actuel, aussi des données diagnostiques: type du processus pathologique, localisation anatomique, l'étiologie et l'activité du processus, le symptome principal. On établit, en même temps, un ou plusieurs effets thérapeutiques escomptés.

Au cours du traitement l'operateur estime l'effet obtenu suite à la séance thérapeutique précédente, en attribuant des coefficients d'accomplissement, ce qui permet la détermination de l'effet thérapeutique global par le système.

b) - Le système affiche sur l'éidophore l'emplacement anatomo-topographique des zones recommandées pour l' application des excitants avec les détails nécessaires et assiste l'operateur dans la procedure de détection des dites zones. La détection est faite interactivement, à l'aide du crayon-détecteur. La détection ainsi faite, le système élimine les zones ayant un niveau d'activité électrocinétique situé au-dessous d'un certain seuil et recommande le placement des électrodes matriciels sur les zones qui vont être excitées en but thérapeutique.

c) - Après avoir placé les électrodes IISE, l'operateur commande la mensuration des zones électrodermiques. Le système mesure l'activité des zones, calcule les derniers paramètres d'excitation et recommande à l' operateur d'écarter les électrodes placés sur les zones de stimulation par radiation cohérente.

d) - Le système affiche sur l'éidophore les sets complets des paramètres d'excitation en champ électrique et en radiation cohérente, établit automatiquement l' emplacement de la paire des lasers et attend l' intervention de l'operateur.

Dans ce moment l'operateur peut être d'accord avec les recommandations du systeme et autoriser l' application des excitations en but thérapeutique. En cas contraire l'operateur peut intervenir en modifiant les paramètres recommandés et exécuter la stimulation avec les paramètre "utilisateur".

e) - Une fois le temps nécessaire pour la stimulation en but thérapeutique est passé, le systéme commande à l'operateur de replacer les électrodes matriciels sur les zones "laser". Ensuite le système commence les nouvelle mensurations de l'activité électrocinétique des zones soumises à l'excitation complexe, calcule et mémorise toutes les données de la séance thérapeutique.

f) - En ce moment la séance thérapeutique est terminée.

Le système BIOTEN guide automatiquement le traitement par la suite d'elaboration successive des décisions, avec lesquelles l'operateur peut être d'accord ou non. L'intervention de l'operateur peut survenir dans n' importe quel moment du traitement sur les recommandations et décisions prises par le système, qui mémorise sur demande tous les changements.

A la fin de chaque étape (séance thérapeutique, intervalle de décision thérapeutique ou fin du traitement) le système élabore une synthèse des données, qui peut être listée sur l'éidophore ou à l'imprimante, sur demande.

Le système peut également fournir, sur demande, une image statistique sur l'ensemble des patients traités.

## 7. DOMAINES D'APPLICATION

Le système BIOTEN s'utilise dans les domaines d' application thérapeutique suivants:
I. Affections où la symptomatologie dominante est algique:

EP 0 473 861 A1

A. La douleur somatique
1. La douleur monoarticulaire
a) La scapulalgie - exemples:
- périarthrite scapulohumérale
- périarthrite douloureuse simple non-ankylosante
- blocage de l'épaule
b) La douleur dans l'articulation du coude
c) La coxalgie
- coxarthrose primitive
- périarthrite coxofémorale
- coxites de spondylite ankylopathique
d) La gonalgie
- gonarthroses primitives
- déchirures et traumatismes ménisquales
e) La tallalgie
- post-traumatique
- bursites retroachilléenes
- syndromes de tunnel tarsien
- spondylite ankylopathique
- ostéoporoses
- ostéophytoses
2. La céphalée
a) Processus intracrânnien non-expansive
- migraine
b) Processus extracrânnien
- nevralgies d'origine: otique, nasale, oculaire ou au niveau cervical (syndrome Barre-Lieau)
3. Les algies non-viscérales de la tête
a) La névralgie trigéminée
b) L'odontalgie
4. Douleur vertébrale
a) La cervicalgie
- arthrose cervicale
- torticolis
b) La nevralgie cervico-brachiale
c) La dorsalgie
- épiphysite vertébrale
- scoliose et cyphose thoracique
- arthrose thoracique
- syndrome trophostatique
- ostéoporose diffuse vertébrale
d) La nevralgie intercostale
e) La lombalgie
- spondylolyse
- spondylolystese
- syndrome trophostatique
- dysarthrose lombaire
- spondylite ankylopathique
- ostéoporose
- lumbago aigue
f) La nevralgie sciatique
- sciatique par hernie discale
- sciatique symptomatique hyperalgique
- sciatique tronculaire post-traumatique
5. Les algies du membre supérieur (autres que la douleur monoarticulaire)
a) Les algies du bras
b) Les algies de l'avant-bras
c) Les algies de la main
d) Les algies des doigts

6. Les algies du membre inférieur (autres que la douleur monoarticulaire)
   a) Les algies de la cuisse
   b) Les algies de la jambe
   c) Les algies de la cheville
7. La douleur polyarticulaire
   - polyartralgie
B. La douleur viacérale
   1. La pleurodynie
   2. L'angor
   3. L'épigastralgie
   4. La colique abdominale diffuse
   5. La colique biliaire
   6. La colique rénale

II. Affections neurologiques
   - parésies
   - paralysies
   - séquelles post-accident vasculaire cérébral
   - la maladie Menière
   - les troubles nerveuses vago-sympathiques
   - les convulsions

III. Les maladies des organs du sens
   - hypo et anosmie
   - otite
   - hypoacusie

IV. Affections médicales fonctionnelles
   - ulcère gastroduodénal
   - asthme bronchique
   - diskinésie biliaire
   - colopathie fonctionnelle chronique
   - dystonie neuro-végétative

V. Affections vasculaires
   - syndrome Reynaud
   - artériopathie périphérique oblitérante
   - oedèmes périphériques

VI. Affections allergiques
   - rhinite allergique
   - urticaire
   - dermatites allergiques

VII. Affections gynécologiques
   - anexite chronique
   - dysménorrhée
   - syndrome prémenstruel
   - impotence et insuffisance sexuelle masculine
   - frigidité féminine

VIII. Affections post-traumatiques
   - toutes sortes

Le système BIOTEN peut être utilisé aussi dans les domaines suivants de la recherche médicale et biologique:
   - la caractérisation de la bioélectrocinétique de l' enveloppe cutanée
   - la caractérisation du contenu d'information de la fonction bioénergétique de l'être vivant
   - des études en ce qui concerne le rapport stimuli/réponse en médicine
   - des études des systèmes de l'intelligence artificielle en médicine

## 8. AVANTAGES

Le système BIOTEN présente les avantages suivants:

1.- Le système est différent par conception des thérapies traditionnelles ou moderne de stimulothérapie ou de réflexothérapie et présente un mode simple, codifié et reproductible d'utilisation pour toutes les catégories des practiciens médicaux; par contre les autres thérapie mentionnées comportent des règles d' application peu communes ou difficiles pour le praticien ordinaire qui n'est pas specialisé particulièrement dans ces domaines.

2.- La reproductibilité parfaite de l'application des excitations électriques et électromagnétiques cohérentes par la commande et le contrôle digital des paramètres.

3.- La possibilité d'obtenir des résultats thérapeutiques différentiés par la combinaison dans diverses proportions des paramètres d'application simultanée des champs.

4.- La reproductibilité accrue d'identification, de sélection et de caractérisation des zones soumi ses à l'application des excitants par un critère unique qui est une proprieté active et dynamique de l'organisme.

5.- La reproductibilité parfaite de la séquence de mesure avant et après l'application des excitants en but thérapeutique.

6.- La possibilité de la génération et du contrôle du phénomène de résonance d'information par l' utilisation d'une interface d'information sensitiveexcitatrice combinée avec le système bi-laser.

7.- L'élaboration automatique des solutions de traitement vers un effet escompté établi initialement par contrôle en boucle de type "biofeedback".

8.- Le traitement appliqué est complètement non-nocif et ne présente aucun effet sécondaire grâce aux faibles niveaux d'énergie appliqué.

9.- Le système peut être utilisé autant dans la recherche fondamentale que dans les domaines cliniquesapplicatifs biomédicaux.

En résumé le système BIOTEN est un ordinateur specialisé destiné à la thérapeutique médicale. Ce système utilise l'application des champs électriques de basse et infrabasse fréquence et des champs électromagnétiques cohérents de très basse puissance, emis par deux sources laser à paramètres d'excitation différents. L'application des champs ainsi générés est effectuée dans le but d' obtenir des effets thérapeutiques différents et initialement escomptés, par l'intermédiaire d'une interface d'information sensitive-excitatrice disposée dans un montage spatial interférentiel au niveau des zones électrodermique actives, préablement sélectionnées et caractérisées en base à des propriétés électrocinétiques precises.

Le système BIOTEN présente une architecture fonctionnelle réalisée par des sous-ensembles d'exe- cution specialisée, coordonnés et contrôlés par un système expert:

- Sous-système specialisé pour mensuration-excitation thérapeutique en champs électriques et électro- magnétiques cohérents, avec des possibilités multiples de réaliser des montages spatiaux interférentiels et de générer le phénomène de résonance d'information.
- Sous-système specialisé pour l'excitation thérapeutique en champs électriques et électromagnétiques cohérents, avec des possibilités multiples de combiner et de contrôler par des moyens digitals les signaux d' excitation.
- Sous-système specialisé bi-laser avec des possibilités multiples de commande et de contrôle différentié de la radiation cohérente appliquée.
- Sous-système specialisé pour mensuration, identification, caractérisation et sélection des zones électrodermiques proposées pour l'application des signaux d'information thérapeutiques.

Le système expert de commande et contrôle des sous-systèmes specialisés est prevu avec des bases de données et de connaissances dynamiques dans lesquelles il y a des sets de paramètres d'application des signaux d'excitation et les zones d'application correspondentes, établis précédemment par des méthodes expérimentales et statistiques. L'individualisation d'application thérapeutique et de la commande de traitement est effectuée en fonction du patient et des données diagnostiques de celui-ci par un algorithme dédié qui réalise en plus la fonction d'assagissement et de spécialisation progressive de l'application thérapeutique.

Du point de vue constructif, le système BIOTEN est un système multiprocesseur, bâti autour d'une architecture d'ordinateur personnel, avec des accessoires standard (clavier, éidophore couleur, disque rigide, disque flexible, imprimante) et muni d'une interface de communication avec des contrôleurs intelligents.

Il y a des contrôleurs specialisés pour les mensurations bioélectrometriques et pour l'application des excitations en champs électriques par l'intermédiaire des électrodes matriciels à double fonction alternative, sensorielle et excitante.

Il y a aussi un contrôleur specialisé pour la commande et le contrôle des deux sources laser pour l'excitation en radiation électromagnétique cohérente et pour mensurations à l'aide d'un crayon-détecteur.

L'architecture du logiciel du système a la composition suivante:

- les 2 bases de données et de connaissances
- le système expert pour la sélection des surfaces cutanées et pour l'élaboration des paramètres d' excitation thérapeutique
- l'interface "utilisateur"
- les modules graphiques
- les sous-programmes de commande et contrôle.

Les domaines d'application thérapeutique sont:

1.- Le traitement des douleurs somatiques et viscérales aiguës ou chroniques.

2.- Le traitement des affections post-traumatiques.

3.- Le traitement de certaines affections neurologiques et neuro-musculaires.

4.- Le traitement de certaines affections rhumatismales chroniques.

5.- Le traitement de certaines affections vasculaires.

6.- Le traitement de certaines affections médicales.

7.- Le traitement de certaines affections allergiques.

8.- Le traitement de certaines affections gynécologiques et sexuelles.

Les domaines d'application dans la recherche fondamentale biomédicale sont:

1.- L'étude de l'électrocinétique de surface.

2.- L'étude du phénomène de résonance d'information.

3.- L'étude du rapport signal-bruit-réponse en biologie.

4.- L'étude des systèmes d'intelligence artificielle en médecine.

Le système BIOTEN présente les avantages suivants:

1.- Le système est différent par conception des thérapies traditionnelles ou modernes de stimulothérapie ou de reflexothérapie et présente un mode simple, codifié et reproductible d'utilisation, pour toutes les catégories de praticiens medicaux; par contre, les autres thérapies mentionnées comportent des règles d'application peu communes ou difficiles pour le praticien ordinaire qui n'est pas specialisé particulièrement dans ces domaines.

2.- La reproductibilité parfaite de l'application des excitations électriques et électromagnétiques cohérentes par la commande et le contrôle digital des paramètres.

3.- La possibilité d'obtenir des résultats thérapeutiques differentiés par la combinaison dans diverses proportions des paramètres d'application simultanée des champs.

4.- La reproductibilité accrue d'identification, de sélection et de caractérisation des zones soumises à l'application des excitants par un critère unique qui est une proprieté active et dynamique de l'organisme.

5.- La reproductibilité parfaite de la séquence de mesure avant et après l'application des excitants en but thérapeutique.

6.- La possibilité de la génération et du contrôle du phénomène de résonance d'information par l'utilisation d'une interface d'information sensitive-excitatrice combinée avec le système bi-laser.

7.- L'élaboration automatique des solutions de traitement vers un effet thérapeutique escompté établi initialement par contrôle en boucle de type "biofeedback".

8.- Le traitement appliqué est complètement non-nocif et ne présente aucun effet sécondaire grâce aux faibles niveaux d'énergie appliques.

9.- Le système peut être utilisé autant dans la recherche fondamentale que dans les domaines cliniquesapplicatifs biomedicaux.


## 9. BIBLIOGRAPHIE

1. Astumian, R.D., Tiang YoW Tsong: Charge-Field in Cell Membranes and Electroconformational Coupling Theory for the Interaction between Dynamic Electric Fields and Membrane Enzymes - International Symposium on Charge and Field Effects in Biosystems, June 4-9, 1989, Virginia Commonwealth University, USA

2. Astumian, R.D. et al.: Proc. Natl. Acad. Sci. USA 82, 434-438, 1987

3. Bertrand, A., Ducassou, D., et al.: Biophysique - Ed. Masson, 1980

4. Bossy, J.: Bases neurobiologiques des reflexothérapies Paris, Ed. Masson, 1975

5. Cleary, S.F., Li-Ming Liu, Guanghui Cao: Functional Alteration of Mammalian Cells by Direct High Frequency Electromagnetic Field Interactions - International Symposium on Charfe and Field Effects in Biosystems, June 4-9, 1989, Virginia Commonwealth University

6. Cleary, S.F. et al.: Abstracts of Eleventh Annual Meeting of the Bioelectromagnetics Society, Tucson, Arizona, June 18-20, 1989

7. Delaney, G.M., Roller, S.D. et al.: Charge and Field Effects in Biosystems - eds. Allen, M.J. and Usherwood, P.N.R. (Abacus Press, Turnbridge Wells, 1984)

8. Eory, A.: Les réports de résistance électrique en fonction des facteurs de corrélation - Thème Doctorat, Budapest, 1973

9. Geddes, L.A., Baker, L.E.: Principles of Applied Biomedical Instrumentation - copyright 1975, a Wiley-Interscience Publication, New York, vol I-II

10. Hyodo, M.: Ryodoraku Treatment - Osaka, 1975

11. Inyushin, V.: Problems of Bioenergetics, Alma Ata, 1969

12. Inyushin, V.: Resonance, Biostimulation and the Problem of Bioplasma - International Symposium of Wave Therapeutics, Paris, 1979

13. Jacobs, N.B.: Neurostimulation Given by Technicians Following Formula Books and Using High-Powered Equipment - Am. J. Acupuncture, 10, 1982

14. Kent, D., Vachtsevanos, G.: A Theoretical Examination of How Power Line Fields Interact with Biological Tissues - International Symposium on Charge and Field Effects in Biosystems, June 4-9, 1989, Virginia Commonwealth University

15. Kenyon, J.N.: Modern Techniques of Acupuncture - Thornsons, Inc., Vol I-II, 1983

16. Kenyon, J.N.: Modern Techniques of Acupuncture - Segmental Electrogram - Vol. III, Chap. VI, Thomson Publishers, 1985

17. Kleinstein, B. (ed.): Biological Effects of Non-Ionizing Electromagnetic Radiation (Abstracts of current literature) - Information Ventures Inc. Philadelphia, 1989

18. Kropp et al., Am. J. Acupuncture, Vol. XIII, No. 3, Sept. 1985, pag. 253-259

19. Lerner, E.J.: Biological Effects of Electromagnetic Fields - I.E.E.E. Spectrum, May, 1984

20. Lavier, I.A.: Bioénergétique chinoise - Paris, Ed. Maleine, 1976

21. Man P.L., Ning T.L.: Elecropunture and Electrostimulation for Relief of Chronic Intractable Pain - Am. J. Acupuncture, 11: 143-147, 1983

22. Mendel, P.: Energetische Terminalpunkt - Diagnose Kosmo-Medizin, 1984

23. Meylan, S.: Bioélectricité - Quelques Problèmes - Paris, Ed. Masson, 1971

24. Michaelski, R.S. et al.: Machine Learning and Artificial Intelligence Approach - Ed. Tioga Publishing Co., 1983

25. Moldovan, C.I.: Multiparametrial Explorations of Some Electro-Dermal Areas in Experimental Cancer and Oncological Clinic - 40th International Congress Ryodoraku, Myazaky, Japan, 8 Oct 1988; 5th International Congress of Oriental Medicine, Seoul, Korea, 7-9 Sept, 1988; 7th International Conference of Psichotronic Research, IAPR conf. cord., Silver Spring, USA, 1989

26. Moldovan, C.I.: Infrared Laser-Stimulotherapy - Preliminary Results - Contributions of Young Researchers in Mechanization, Automatization and Electronization of the National Economy - 2nd Symposium, Politechnical Institute Bucharest, 20 Oct 1989

27. Moldovan, C.I.: considerations for Optimization of a Computerized Biostimulotherapy Equipped with a Thermovision Apparatus - Contributions of Young Researchers in Mechanization, Automatization and Electronization of the National Economy - 2nd Symposium,Politechnical Institute Bucharest, 20 Oct 1989

28. Moldovan, C.I,: Registering Electro-Thermo-Dermal Method (ETD) of Some Cutaneous Active Areas. Application in Onchological Field - International Acupuncture Symposium, Bucharest, 1986.

29. Moldovan, C.I.: Electro-Acupuncture Treatment Method for Superior Limb Oedema Secondary to Radio-Surgical Treatment in Female Mamary Neo-plasm - International Medical Acupuncture Conference, London, May 4-8th 1986; American Journal of Acupuncture, vol. 15, no. 2 April-June 1987

30. Moss, T.: The Body Electric - Ed. J.P. Tarcher Inc., 1979

31. Nakatami, Y.: Neurometer Demonstration Documents, 1969

32. Nakatami, Y.: Skin Electric Resistance and Ryodoraku - J. Annonomic Nerve 6: 52, 1956

33. Niboyet, J.E.H.: La moindre résistance à l' électricité des surfaces punctiformes et des trajets cutanés concordants avec les points et méridiens bases de l'acuponcture - Imp. Louis-Jean, Lyon, 1963

34. Presta, E., Wang, J., et al.: Measurement of Total Body Electrical Conductivity: a New Method for Estimation of Body Composition - Am. J. Clin. Nutr., 37: 735-739, 1983

35. Ross, W.A.: The extracellular Space and Energetic Hierarchies in Electrochemical Signalling Between Cells - International Symposium on Charge and Field Effects in Biosystems, June 4-9, 1989, Virginia Commonwealth University

36. Spikes, J.D.: Photobiology, Rd. Giese, A.C., Acad. Press, N.Y., 1986

37. Tiller, W.A.: Explorations of Electrodermal Diagnostic and Treatment Instrument, J. Holystic Medicine, 4 (2), 105-127, 1988

38. Tremolière, J.: La neurostimulation dans le traitement de la douleur - Rev. Electronique Applications, No. 21, 1982

39. Ullman, D.: Conceptualizing Energetic Medicine: A Beginning - Am. J. Acupuncture, 9, 1981

40. ***: Laser Photobiology and Photomedicine, Proceedings of a two-week course on laser applications to biology and medicine, Plenum Press, N.Y., 1985

41. ***: The Phenomenon of Medicine Testing in Electroacupuncture According to Voll. Am. J. Acupuncture, 8:97,1980.

**Revendications**

1. Procédé pour le traitement d'information en champ électrique et électromagnétique caractérisé en ce qu' on utilise l'application simultanée et contrôlée du champ électrique de basse et infra-basse fréquence et de la radiation électromagnétique cohérente emise par deux sources laser de faible puissance et paramètres d'excitation différents; l'établissement de ces paramètres et le contrôle de ces applications s' effectuant dans la séquence suivante:

- on identifie et on effectue une sélection successive des zones électrodermiques proposées initialement pour la stimulation en déterminant un paramètre dépendant du potentiel et de la résistance électrique cutanée, mesurée par une mfhode atypique, en utilisant une chaîne de mensuration avec une impedance d'entrée comparable à celle du tégument, défini comme coefficient de transfert électrocinétique et représentant la mesure de l' activité électrocinétique, evaluée sur une échelle arbitraire; la sélection des dites zones est faite par rapport à un seuil individuel établi dans le moment de l'exploration par rapport aux variations de l'électrocinétique locale; suite à la sélection on obtient un set de zones électrodermiques actives plus petit ou égal au set initial recommandé;

- on propose un set de paramètres d'application du champ électrique: le montage spatial des paires d' électrodes, le train et les modalités de génération des impulsions, la forme des impulsions, le montage interférentiel résultant de la combinaison du train et des modalités de génération des impulsions avec le montage spatial des paires d'électrodes; ce set est developpé d'un set initialement établi par des méthodes expérimentales et statistiques;

- dans les zones sélectionnées dans le montage spatial recommandé on place successivement les électrodes matriciels à double fonction alternative, sensorielle et excitante et on etablit les coefficients de transfert électrocinétique pour chaque surface élémentaire;

- on détermine l'activité électrocinétique de surface des zones sélectionnées en calculant le gradient électrocinétique local, représentant la distribution des coefficients de transfert électrocinétique pour la surface cutanée ainsi mesurée et le gradient électrocinétique global qui représente la mesure de l'activité étectrocinétique distribuée au niveau des zones sélectionnées;

- on établit automatiquement la polarité des électrodes matriciels pour les zones sélectionnées, séparement pour les zones destinées pour l' excitation en champ électrique et celles pour l' excitation en radiation cohérente: on cherche la zone caractérisée par un gradient électrocinétique maximal en attribuant la polarité negative et on attribue la polarité positive à la zone paire.

- on propose deux sets différents des paramètres d' application de la radiation électromagnétique cohérente: la longeur d'onde, la puissance, la fréquence des trains d'impulsions et les modalités d'application, variables en fonction des données expérimentales et statistiques; on atribue au premier set les zones de polarité negative, en écartant les électrodes matriciels correspondants.

- on applique le champ électrique pour un intervalle de temps préétabli et la radiation cohérente pour un intervalle de temps plus petit ou égal au premier; suite à l'application simultanée du champ électrique et électromagnétique aux paramètres individualisés et en fonction de l'activité électrodynamique des zones soumises à la stimulation conformément à la méthode, se produisent au niveau des zones d' application des effets bioélectroniques, biochimiques, de champ électromagnétique et de résonance d'information; l'amplification de ces effets locaux conduit à l'apparition des effets systèmiques par l'action des neuromédiateurs, des substances biologiques actives, des hormones, des mécanismes de contrôle au niveau médullaire et central etc.; l'application des champs électriques et électromagnétiques conduit aux effets locaux et systèmiques différents, en fonction de la modulation de ceux-ci; après l'excitation on mesure un nouveau set des coefficients de transfert électrocinfique, on calcule un nouveau set des gradients électrocinétiques locaux et on calcule l'indice global de l'effet de l'excitation sur l'activité électrocinétique;

- les dites données: les variables individuelles, les sets des zones et les paramètres d'application initialement recommandés, les sets des zones sélectionnées, l'evolution des polarités, des paramètres d'application des excitants et l'indice global de l'effet de l'excitation sur l'activité électrocinétique, qui représente la mesure et l' évolution de la résonance d'information du

20

traitement sont analysées par des méthodes statistiques et euristiques; suite à l'analyse on peut déterminer des sets optimals initials d' excitation électrique et électromagnfique cohérente.

2. Interface d'information sensorielle excitatrice pour la mise en oeuvre du procédé de la révendication 1 , caractérisée en ce que, dans le but d'obtenir un échange d'ingormation/énergie d'un part et dd l'autre d'une surface de séparation et la génération du phénomène de résonance d'information dans une biostructure, on utilise un set d'électrodes matriciels à double fonction alternative, de mesure et d'excitation en champ électrique, interconnecté avec un bloc d'analyse des données d'entrée et un bloc de commande des signaux de sortie; l'électrode matriciel est une surface de mesure/stimulation sur laquelle sont disposés un nombre d'électrodes de contact.

3. Appareillage pour le traitement d'information en champ électrique et électromagnétique, selon le procédé de la révendication 1 , incorporant le dispositif de la révendication 2 , caractérisé en ce qu'il est constitué d'un système multiprocesseur (fig. 3) et bâti autour d'une architecture d'ordinateur personnel (OP) avec des accessoires standard - clavier, éidophore couleur, disque rigide, disque flexible, imprimante - et muni d'une interface de communication avec les contrôleurs intelligents (CSE), (CSL); les contrôleurs (CSE) (fig. 4) sont specialisés pour la stimulation électrique - champ électrique de basse et infra-basse fréquence - et aussi pour l'acquisition des données par l'intermédiaire des électrodes matriciels à double fonction, sensorielle et excitante, alternative (fig. 5); le contrôleur (CSL) (fig. 6) est specialisé pour la stimulation en champ électromagnétique par radiation cohérente, en utilisant dans ce but des lasers de faible puissance (L1, L2) et doté d'un crayon-détecteur connecté avec une chaîne d'acquisition pour les mensurations dans la phase de sélection des zones cutanées.

4. Appareillage pour le traitement d'information en champ électrique et électromagnétique pour la mise en oeuvre du procédé selon la révendication 1 , utilisant le dispositif de la révendication 2 selon la révendication 3 , caractérisé en ce qu'il est bâti autour d'une architecture d'ordinateur personnel (OP) avec des accessoires standard (clavier, éidophore couleur, disque rigide, disque flexible, imprimante) et muni d'une interface de communication avec les contrôleurs intelligents (CSE), (CSL); le logiciel de système qui tourne sur cet ordinateur consistant dans les deux bases de données et de connaissances, du système expert pour la sélection des zones cutanées d' interêt et pour l'élaboration des paramètres d' excitation, des modules de calcul statistique, des modules graphiques, des interfaces "utilisateur" et de communication avec les autres contrôleurs intelligents du système; les autres ressources de calcul (acquisition, commende et contrôle des paramétres d' excitation etc.) étant distribuées parmi les contrôleurs (CSE), (CSL).

5. Appareillage pour le traitement d'information en champ électrique et électromagnétique, selon les révendications 2 et 3 , caractérisé en ce que les contrôleurs (CSE) sont specialisés pour la stimulation électrique - champ électrique de basse et infra-basse fréquence - et aussi pour l'acquisition des données; l'interface de mesure et d'excitation se fait par l' intermédiaire des électrodes matriciels (EM) à double fonction, sensorielle et excitatrice, alternative; le contrôleur (CSL) est specialisé pour la stimulation en champ électromagnétique par radiation cohérente, en utilisant dans ce but les lasers (L1) et (L2), pour les mensurations dans la phase de sélection des zones cutanées; le dit contrôleur est doté d'une chaîne d' acquisition et est muni d'un crayon-détecteur (CD).

6. Appareillage pour le traitement d'information en champ électrique et électromagnétique, incorporant le dispositif selon le révendication 2 et selon les révendications 3 et 4 , caractérisé en ce que le contrôleur (CSE) (fig. 4) contient un microcontrôleur (MCU), qui surveille le fonctionnement des chaînes d' acquisition et de stimulation et des autres circuits; la communication avec le processeur "master" du système se faisant par l'intermédiaire du circuit d' interface (I); le bloc d'interface avec l'utilisateur (BIU) étant responsable de la communication avec l' operateur; le microcontrôleur (MCU) comportant aussi des circuits "timer" pour la réalisation d'une horloge de temps réel; le générateur programmable (GEN) produisant des impulsions de commande à fréquence, durée et mode de travail programmable; le signal résultant et le signal provenant du circuit convertisseur digital-analogique (D/A) commandant le bloc générateur d'impulsions (BGI), qui forme les impulsions de stimulation en champ électrique de fréquence comprise dans le domaine 1....1000 Hz, ces impulsions étant distribuées par l'intermédiaire du bloc commutateur (BK) aux 3 paires d'électrodes matriciels à fonction excitatrice (EM); le bloc (BK) permettant également de connecter un des électrodes matriciels à l'entrée du chaîne d'acquisition des données; cette chaîne étant formée d'un bloc d' amplification et conditionnement des signaux (BAC),

où les signaux prélevés par les électrodes matriciels à fonction sensorielle sont convertis et amplifiés, du circuit multiplexeur (MUX) et du convertisseur analogique-digital (A/D), le tout commendé par le bloc de contrôle (BC).

7. Utilisation de l'appareillage selon les révendications de 3 à 6 dans les installations médicales en but diagnostique et thérapeutique, notamment pour les affections algiques, neurologiques, fonctionelles, des organes des sens, vasculaires, allergiques, gynécologiques et post-traumatiques.

8. Utilisation de l'appareillage selon les révendications de 3 à 6 dans les installations pour l'étude de l' électrocinétique de surface.

9. Utilisation de l'appareillage selon les révendications de 3 à 6 dans les installations pour l'étude du rapport signal-bruit-réponse en biologie.

10. Utilisation de l'appareillage selon les révendications de 3 à 6 dans les installations pour l'étude des systèmes d'intelligence artificielle.

START: k=1

a) Les parametres d'etat / du
   Les donnees diagnostiques / malade

b) On etabli les effets
   therapeutiques escomptes

c) On recomande le set des zones paires: z1 + z2

Evalue l'effet therapeutique
cumule ETCk obtenu sur IDT

d) On mesure et on fait la selection par rapport au seuil
   M < z1+z2 zones

*) Evalue l'effect therapeutique
   global ETG obtenu apres la
   derniere seance therapeutique

e) On recomande un set des parametres d'application des champs electrique et
   electromagnetique

f), g) On mesure et on determine le degre d'activite et la distribution de
       l'activite electrocinetique sur les zones selectionnees

h), i) On etabli la polarite des paires des electrodes sur les zones selectionnees
       et les parametres d'application des champs electrique et electromagnetique
       (E), (L1), (L2) = f(a, b, d, e, f, g, *)

j) On applique le champ electrique (E) - un interval de temps: TE
   On applique le champ electromagnetique (L1),(L2) - un interval de temps: TL < TE

k) On determine l'effet de l'application des excitations sur l'activite electrocinetique des zones selectionnees

k<-k+1

oui        IDT        non
           fini

oui     k=Max     non

non     ETCk=[4     oui

CONTINUE ((E),(L1),(L2))     oui

non

REEVALUATION ((E),(L1),(L2))     oui

non

( STOP )    (STOP / MEMORIZE)    ( STOP )

Fig. 1

Fig.2

24

EP 0 473 861 A1

Fig. 3

Fig. 4

25

Fig. 5

Fig. 6

26

Office européen
des brevets

**RAPPORT DE RECHERCHE
EUROPEENNE**

Numéro de la demande

**EP 90 81 0663**

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | FR-A-2 589 067  (G. BERNAZ)<br>* le document en entier *<br>— — — | 1,5,7 | A 61 N 1/40<br>A 61 N 1/18<br>A 61 N 1/08<br>A 61 N 5/06 |
| A | WO-A-8 700 760  (BBC-SECHERON S.A.)<br>* abrégé * * page 7, ligne 13 - page 13, ligne 10 * * page 14, ligne 19 - page 18, ligne 11; figures *<br>— — — | 1-7 | |
| A | DE-A-2 740 969  (K.H. CASPERS)<br>* le document en entier *<br>— — — | 1,2,7 | |
| A | DE-A-3 900 428  (H. HORA)<br>* le document en entier *<br>— — — | 1,3-7 | |
| A | FR-A-2 441 294  (G.R. BRETTE)<br>* le document en entier *<br>— — — | 1,7 | |
| A | US-A-4 505 275  (WU CHEN)<br>* abrégé; figure 1 *<br>— — — — — | 1 | |

| DOMAINES TECHNIQUES RECHERCHES (Int. Cl.5) |
|---|
| A 61 N<br>A 61 H |

Le présent rapport de recherche a été établi pour toutes les revendications

| Lieu de la recherche | Date d'achèvement de la recherche | Examinateur |
|---|---|---|
| La Haye | 10 mai 91 | FERRIGNO, A. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire
T : théorie ou principe à la base de l'invention

E : document de brevet antérieur, mais publié à la date de dépôt ou après cette date
D : cité dans la demande
L : cité pour d'autres raisons
........................................................
& : membre de la même famille, document correspondant